Europäisches Patentamt

**European Patent Office**

Office européen des brevets

(11) Publication number: **0 074 055**
**A2**

# (12) EUROPEAN PATENT APPLICATION

(21) Application number: **82107979.5**

(22) Date of filing: **31.08.82**

(51) Int. Cl.³: **A 61 B 5/02**
G 01 L 3/00

(30) Priority: **03.09.81 US 298972**

(43) Date of publication of application:
**16.03.83 Bulletin 83/11**

(84) Designated Contracting States:
**DE FR GB IT NL**

(71) Applicant: HONEYWELL INC.
Honeywell Plaza
Minneapolis Minnesota 55408(US)

(72) Inventor: Aagard, Roger L.
6738 Third Avenue South
Richfield Minnesota 55423(US)

(74) Representative: Rentzsch, Heinz et al,
Honeywell Europe S.A. Holding KG Patent- und
Lizenzabteilung Kaiserleistrasse 55
D-6050 Offenbach am Main(DE)

(54) Fiber optic pressure sensor.

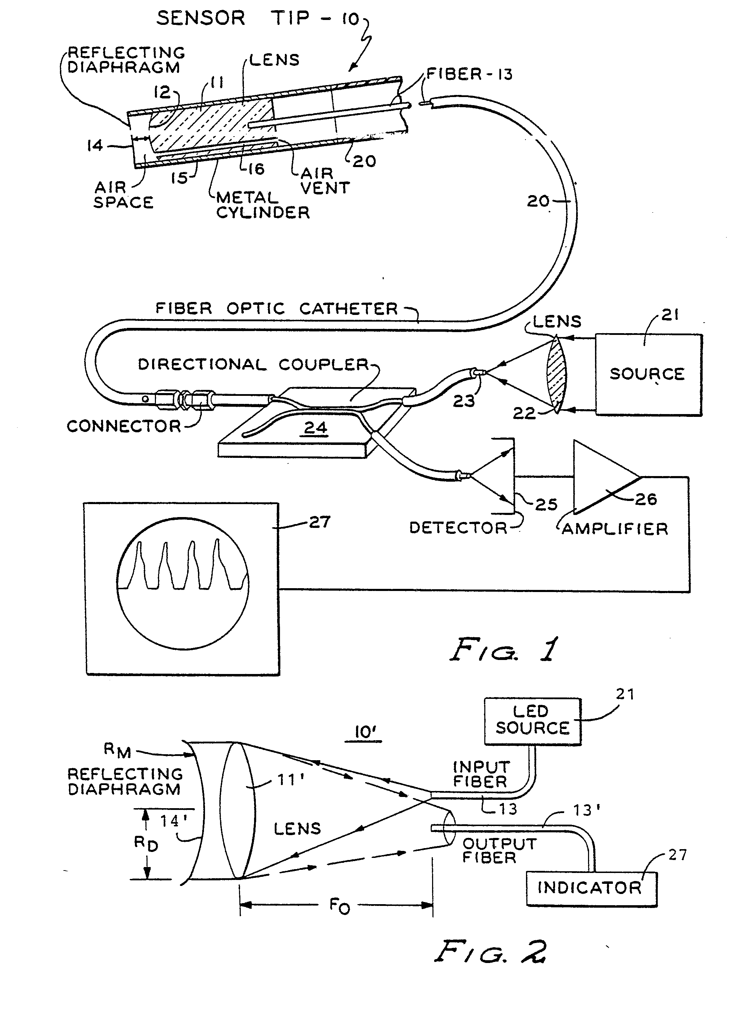

(57) A fiber optic pressure sensor apparatus, in particular an intravascular catheter tip fiber optic pressure sensor is disclosed, wherein a lens element (11) at the end of the fiber optic (13) collimates the light emanating from the fiber and directs the light in a column towards a closely spaced reflecting diaphragm (14). The diaphragm is pressure responsive and modulates the focal length of the lens-diaphragm-mirror combination (Figure 1).

Fig. 1

Croydon Printing Company Ltd.

HONEYWELL INC.                                    August 31, 1982
Honeywell Plaza            ·                       B2009225 EP
Minneapolis, Minn. USA                            HZ/ep

## Fiber Optic Pressure Sensor

## Background and Summary of the Invention

The present invention relates to a fiber optic pressure sensor according to the preamble of claim 1. Such a pressure sensor preferably is used in the field of measuring of intravascular blood pressure.

Measurements of intravascular blood pressure are usually performed with a hollow catheter tube filled with saline solution and attached to an external transducer. This has the drawback of poor frequency response due to the long fluid column. In addition, saline solution is a poor dielectric, making electrical shock hazards a concern.

Catheter tip sensors have been proposed in which light from one or more fibers in a bundle is reflected off a mirrored diaphragm and back into different optical fibers. Pressure induced motion of the diaphragm modulates the intensity of the returned light which is then measured to infer pressure. One such pressure transducer is disclosed in U.S. patents 3,503,116 and 3,580,082.

Departing from said known fiber optic pressure sensor, it is the object of the present invention to improve its performance and reliability. This object is achieved according to the characterizing features of claim 1. Further advantageous embodiments of the present invention may be taken from the subclaims.

In the present invention a fiber optic pressure sensor is described in which a reflecting diaphragm modulates the focal length of a lens-mirror combination. A pressure deformable single reflecting curved diaphragm is used as an optical element together with a lens or refracting element on the optical fiber end. The lens element collimates the cone of light emanating from the fiber and directs the light in a column towards the reflecting diaphragm, the diaphragm being separated by a very small distance from the lens. This refracting element on the end of the

-2-

optic fiber preferably is a molded optical plastic lens such as a lucite member with a spherically curved surface.

## BRIEF DESCRIPTION OF THE DRAWINGS

Figure 1 is a partial block and partial schematic diagram of one embodiment of the fiber optic pressure sensor according to the invention.

Figure 2 is a schematic representation of another embodiment of the fiber optic pressure sensor.

## DETAILED DESCRIPTION

Referring now to Figure 1 there is shown in somewhat schematic form a single fiber embodiment of the fiber optic pressure sensor apparatus commencing with a catheter sensing tip 10. The sensing tip 10 comprises a molded optical plastic lens 11 having a spherically curved surface 12 at the face opposite that into which the optic fiber 13 is fastened. The tip also comprises a thin polyester reflecting diaphragm 14 which is spaced and fastened a small distance away from the lens by a cylinder member 15. The diaphragm is pressure responsive. An air vent 16 in the form of a small hole through the lens allows the pressure to equalize with the atmosphere on the fiber side. The lens 11 is specifically designed to collimate or nearly collimate the cone of light emanating from the fiber 13 and to direct the light to the reflecting diaphragm 14. Although the lens is described above as a conventional molded optical plastic lens, no such limitation is intended and the lens may be of other types such as a graded index lens, often called GRIN or Selfoc, for example. The pressure deformable mirror diaphragm cooperates with the lens as a lens-mirror-lens optical system, with the diaphragm modulating the effective focal length of the lens-mirror combination. The diaphragm is shown in a deformed position resulting from pressure against the outside, with no pressure the diaphragm will be more flat.

The sensing tip is affixed at the end of a catheter 20. The sensor tip is of the same diameter as the catheter and the optical fiber 13 is small in diameter and fits inside the hollow catheter. The catheter 20 near the connector has an aperture for communicating atmospheric pressure to the inside of diaphragm 14. The sensing tip portion 10 of the pressure catheter is shown much enlarged in Figure 1 for purposes of clarity in explanation. A light source 21 and suitable lens 22 focuses the light into the other end 23 of the fiber optic. The light source 21 may also be a solid state light source which abuts directly against the end of the fiber. A conventional directional coupler 24 transmits the light from the source to the fiber 13 and channels the reflected light to a detector 25. The electrical signal processing electronics such as an amplifier 26 and display 27 receive the signals from the detector 25 for converting the optical signal to an electrical signal which can be further conditioned, displayed or recorded.

In the undistorted position of the mirror diaphragm the fiber end is imaged onto itself with unit magnification. When the mirror diaphragm is distorted by pressure, the radiation is spread to cover a larger area, that is, the focal length of the lens-mirror combination is changed. Thus the amount of reflected light collected by the fiber varies as a function of the pressure.

Figure 2 discloses diagrammatically another embodiment of the sensing head 10' in which a dual optic fiber, that is, an input fiber 13 and an output fiber 13', is fastened to the lens 11'. Light emanating from the input fiber 13 is collimated by the lens 11' towards the reflecting diaphragm 14'. The reflected light is refocused by the lens 11' toward the output fiber 13'. Pressure applied to the diaphragm 14' produces a spherical curvature of radius

-4-

R which causes the image to be magnified and focussed beyond the end of the output fiber 13'. The Figure 2 modification of the sensing head is currently the preferred embodiment because it is more amenable to reproducibility.

While the reflecting diaphragm has been indicated above as being formed of polyester film, other diaphragm materials have been tested including stainless steel, aluminum and brass.

Claims:

1. Fiber optic pressure sensor apparatus, in particular intravascular blood pressure catheter, comprising a light source, fiber optic means, a pressure responsive reflecting diaphragm and means for detecting the reflected light, c h a r a c t e r i z e d  b y light-collimating lens means (11, 11') at the end of the fiber optic means (13, 13') and positioned for directing a column of light to the pressure-deformable reflecting diaphragm (14, 14'), with said diaphragm when being un-deformed reflecting maximum light back to said lens means and when being distorted by increasing pressure reflecting less of the light in a direction to fall on said lens means.

2. Sensor according to claim 1, c h a r a c t e r i z e d i n  t h a t  said fiber optic means comprises a transmit (13) and a return (13') optic fiber.

3. Sensor according to claim 1 or 2, c h a r a c t e r - i z e d  i n  t h a t  the fiber optic means (13, 13') extends into a sensor tip (10, 10') including the pressure deformable diaphragm (14, 14') and the light-collimating lens means (11, 11').

4. Sensor according to claim 3, c h a r a c t e r i z e d i n  t h a t  said sensor tip (10, 10') is hollow and comprises a sealed air chamber between said diaphragm (14, 14') and said lens means (11, 11').

5. Sensor according to claim 4, c h a r a c t e r i z e d b y  an air passage (16) through said lens means (11) so that ambient atmospheric pressure can be communicated to said chamber.

SENSOR TIP - 10

REFLECTING DIAPHRAGM

LENS

FIBER - 13

12   11

14

15   16

AIR SPACE

METAL CYLINDER

AIR VENT

20

20

FIBER OPTIC CATHETER

DIRECTIONAL COUPLER

LENS

21

SOURCE

CONNECTOR

24

23

22

DETECTOR

25

AMPLIFIER

26

27

*Fig. 1*

10'

LED SOURCE

21

$R_M$

REFLECTING DIAPHRAGM

11'

INPUT FIBER

13

13'

14'

$R_D$

LENS

OUTPUT FIBER

INDICATOR

27

$F_O$

*Fig. 2*